# EUROPEAN PATENT APPLICATION

(11) **EP 2 743 264 A1**
(43) Date of publication of application: **18.06.2014**
(21) Application number: 12197049.5
(22) Date of filing: 13.12.2012
(51) Int. Cl.: C07D 305/14, A61K 31/337, A61P 35/00

(54) **New crystalline form of cabazitaxel, process for the preparation and pharmaceutical compositions thereof**

(71) Applicant: INDENA S.p.A., 20139 Milano (IT)
(72) Inventor: Cabri, Walter, 20139 MILANO (IT); Ciceri, Daniele, 20139 MILANO (IT); Domenighini, Luca, 20139 MILANO (IT); Gambini, Andrea, 20139 MILANO (IT); Peterlongo, Federico, 20139 MILANO (IT)
(74) Representative: Minoja, Fabrizio

(57) **Abstract**

The present invention relates to a new crystalline form of Cabazitaxel, herein designated as form G, obtained by crystallization of Cabazitaxel from acetonitrile or from mixtures of acetonitrile and water. The new crystalline form G of the invention is endowed with several advantageous properties as compared to the previously disclosed forms of Cabazitaxel.

## Description

### FIELD OF THE INVENTION

The present invention relates to a new crystalline form of Cabazitaxel, to a process for the preparation and to pharmaceutical compositions thereof.

### BACKGROUND OF THE INVENTION

Cabazitaxel is a semi-synthetic derivative of the natural taxoid 10-deacetylbaccatin III, commercialized as acetone solvate. It does stabilize microtubules leading eventually to the mitotic arrest of proliferating cells. It has been approved in the United States of America for the second line treatment of hormone-refractory prostate cancer following a docetaxel-based treatment.

Cabazitaxel has the following formula (I):

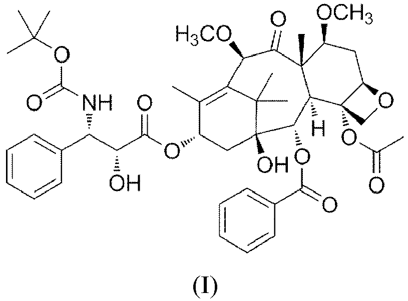

Its chemical name is 4α-acetoxy-2α-benzoyloxy-5β,20-epoxy-1β-hydroxy-7β,10β-dimethoxy-9-oxo-11-taxen-13α-yl (2R,3 S)-3-tert-butoxycarbonylamino-2-hydroxy-3-phenylpropionate.

Cabazitaxel and methods for the preparation thereof are described in WO96/30355 and in WO99/25704.

WO2005/028462 describes an acetone solvate of Cabazitaxel, sometimes referred to as form A. Despite the fact that crystallisation of the acetone solvate is a very effective way for removing impurities, a better pharmaceutical form will be pure Cabazitaxel without any crystallization solvent.

Additional crystalline solvate forms of Cabazitaxel referred to as form I (toluene solvate), form II (methyl tert-butyl ether solvate), form III (2-propanol solvate), form IV (1-butanol solvate), form V (1-propanol solvate) and an amorphous form of Cabazitaxel in a powdery, non-foamy form are described in WO2012/142117. Solvates are rarely use in pharmaceuticals because the solvents are volatile thus making it difficult to maintain the solvent in the crystal. If the API desolvates due to storage conditions or otherwise, it could lead to the formation of multiple polymorphs with different physical properties. Additionally, amorphous solids are metastable and can lead with time to the formation of different polymorphs with different physical properties.

WO2009/115655 discloses five anhydrous forms of the compound, referred to as forms B, C, D, E and F; three ethanol solvates, referred to as ethanolate forms B, D, E; an ethanol/water heterosolvate form F; and a monohydrate-solvent free form C and a dihydrate-solvent free form C. Reaching high purities with these forms is only possible providing the API has been previously purified by other techniques such as for example passing through the acetone solvate (as described in the application). However the introduction of a further purification technique hampers the manufacturing process with inefficiency due to longer production times and lower yield.

WO2009/11565 discloses two hydrate forms of the compound in particular mono and di-hydrate, both hydrate forms are obtained from anhydrous form C by exposition to moisture. The anhydrous form C as described above is obtained in high purity only by passing through the acetone solvate.

A crystalline form of Cabazitaxel obtained from acetone/water is described in CN 102675257 A.

It will then be desirable to find a new crystalline form able to solve the aforementioned problems.

### BRIEF DESCRIPTION OF THE INVENTION

Surprisingly, it has now been found that, crystallising Cabazitaxel using acetonitrile or acetonitrile in mixture with water provides a new solid form, herein designated as form G, with high purity (HPLC: any impurity not more than 0.1 %) and devoid of organic solvents. A further advantage of form G is that it allows to obtain Cabazitaxel in high purity directly without any other form of purification (i.e. prior crystallisation as acetone solvate). Form G can also be obtained from any other known crystalline form.

Ease of isolation by filtration or centrifugation compared to the acetone solvate is a further asset of form G.

### BRIEF DESCRIPTION OF THE FIGURES

- Figure 1: X-RPD pattern of the crystalline form G of Cabazitaxel

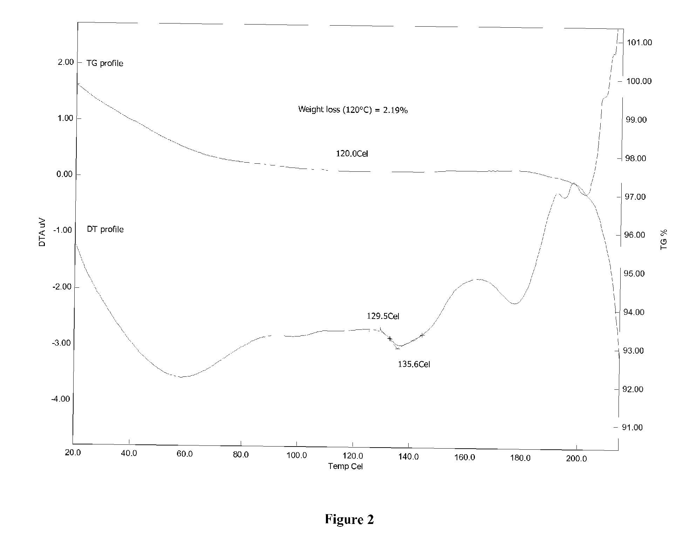

- Figure 2: TG and DTA profiles of the crystalline form G of Cabazitaxel

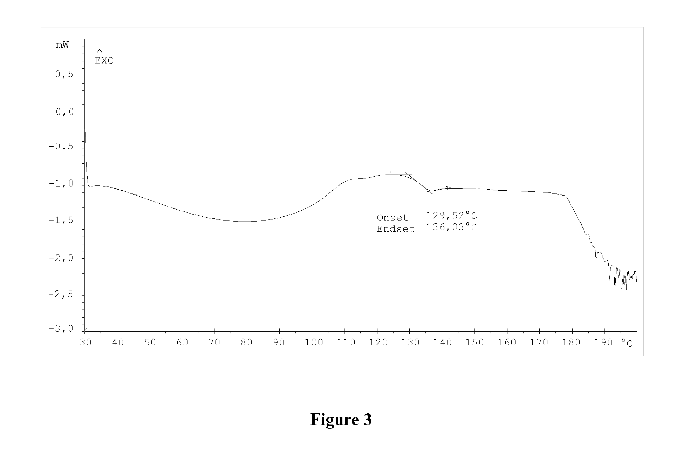

- Figure 3: DSC profile of the crystalline form G of Cabazitaxel
- Figure 4: FTIR spectrum of the crystalline form G of Cabazitaxel in the 4000-550 cm⁻¹ spectral range
- Figure 5: Kinetic of moisture sorption at 25°C of the form G of Cabazitaxel
- Figure 6: Isotherm plot at 25°C for of the form G of Cabazitaxel

### DETAILED DESCRIPTION OF THE INVENTION

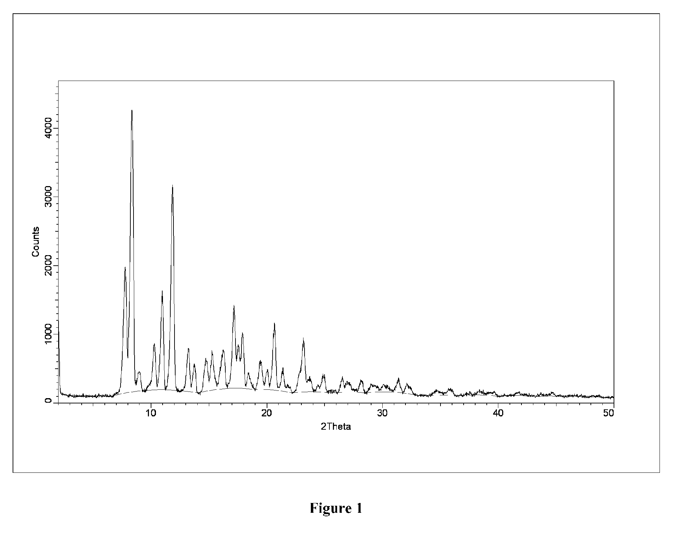

Crystalline form G of Cabazitaxel according to the present invention is characterised by a x-ray powder diffraction (X-RPD) pattern substantially as depicted in Figure 1. The X-RPD pattern shows a crystalline structure and comprises distinctive reflections, expressed as 2-theta degrees values, at approximately 7.6, 8.1, 8.8, 9.7, 10.1, 10.8, 11.6, 13.0, 13.5, 14.6, 15.1 and 16.0 ± 0.2.

Form G shows a water content ranging from 0% to 7% (Karl Fischer titration) depending on the degree of relative humidity it has undergone. This corresponds to a range from 0 to 3 water molecules. Independently from water content 0-7% (Karl Fischer titration) all the potential hydrate forms are isomorphs showing the same X-ray powder diffraction spectrum. The X-ray powder diffraction of the wet crystals obtained by either pure acetonitrile or water acetonitrile are identifical to form G. We can not exclude that a metastable solvate acetonitrile form is initially filtered off.

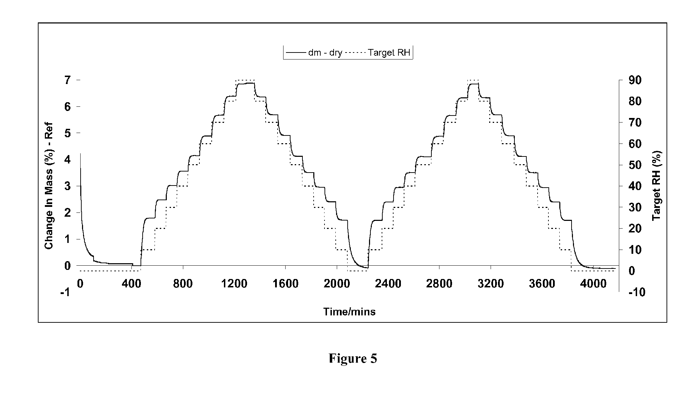

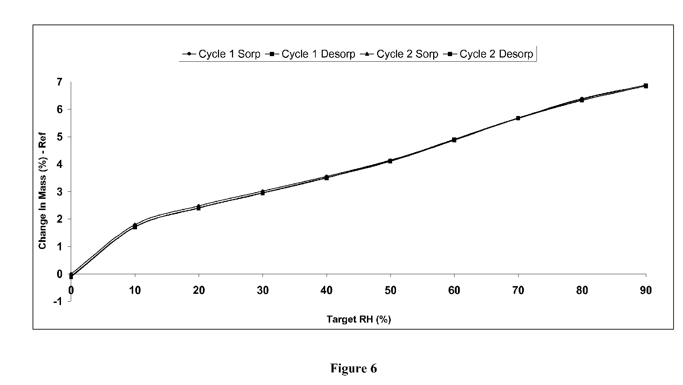

Crystalline form G of the invention may be further characterised by Dynamic Vapour Sorption (DVS) plots substantially as depicted in Figures 5 and 6.

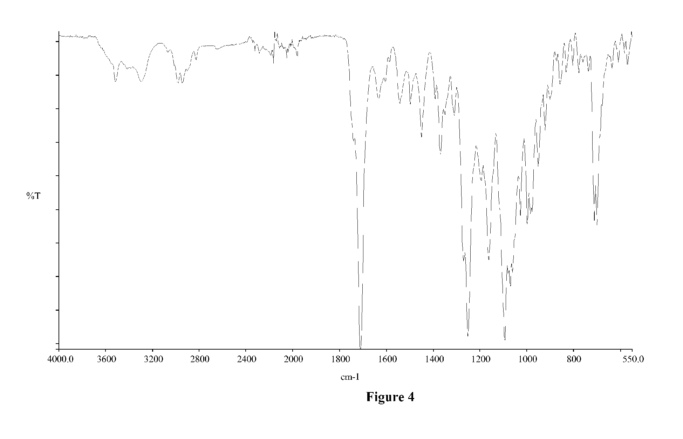

Crystalline form G of the invention may be further characterised by a Fourier-Tranform InfraRed Spectroscopy (FTIR). Spectrum in the 4000-550 cm⁻¹ spectral range substantially is depicted in Figure 4. In particular, the anhydrous form G of the invention shows characteristic absorption frequencies at approximately 3517, 3298, 2983, 2948, 2828, 1712, 1654, 1543, 1498, 1451, 1393, 1369, 1270, 1252, 1163, 1095, 1071, 712 and 700 cm⁻¹.

When the crystalline form G of Cabazitaxel according to the present invention is referred to herein as being characterized by graphical data substantially as depicted in a figure, such as for, for example, the XPRD diffractogram, the TG/DTA and DSC profiles, the FTIR spectrum, the DVS plot, the skilled person will understand that such graphical representations of data may be affected by small variations which may be triggered by experimental variability affecting the instrumental response and/or the sample concentration and purity. These variations are well known to the skilled person and they will not prevent him from comparing the graphical data in the figures herein with graphical data generated for an unknown crystal form and from assessing whether the two sets of graphical data are characterizing the same crystal form or two different crystal forms.

Form G of the invention may be obtained with the content of each impurity lower than 0.1% and a purity higher than 99% when obtained by crystallization from acetonitrile or from mixtures of acetonitrile and water.

According to one embodiment, the crystalline form G of the invention is prepared as described in the examples 1, 2, 3, 4 and 5 by acetonitrile or acetonitrile/water crystallization. Specifically, the process for the preparation of the crystalline form G of the invention comprises the following steps:
a) dissolution of Cabazitaxel in acetonitrile or acetonitrile/water at a temperature higher than 30°C;
b) decreasing the temperature to crystallize Cabazitaxel;
c) filtration and drying under vacuum to afford the crystalline form G.

The crystalline form G of the invention is endowed with several advantageous properties as compared to the previously disclosed forms of Cabazitaxel in term of, for example, high purity obtainable without the need of an additional crystallisation, stability to conversion to other polymorphic forms irrespectively to the water content, better handling and improve processability.

In view of the above described advantages, the form G of Cabazitaxel is useful for the preparation of Cabazitaxel, Cabazitaxel salts, and polymorphic forms thereof.

The form G of the invention is particularly useful as a medicament, especially for the treatment cancers and, in particular, of prostate cancers such as, for example, hormone-resistant prostate cancer.

The above uses of the crystalline form G of Cabazitaxel represent a further object of the invention.

For the therapeutic uses, crystalline form G of the invention may be incorporated in standard pharmaceutical compositions containing at least an excipient suitable for the pharmaceutical uses, which represent a further object of the invention.

The invention is now further illustrated by the following examples.

### EXAMPLE 1

The solvent of reaction mixture of the last step of the synthetic route reported in W02012/142117 containing crude Cabazitaxel, was evaporated and substituted with acetonitrile. The solution was left to crystallize at room temperature. The precipitate was filtered, washed and dried under vacuum. Crystalline form G of Cabazitaxel with all impurities below 0.10% (HPLC analysis) was obtained, yield 85%.

### EXAMPLE 2

Cabazitaxel acetone solvate obtained as reported in WO 2005/028462 was dissolved in CH₃CN at temperature higher than 30°C. Subsequent to complete dissolution, the temperature was decreased to 20-25°C and the product was left to crystallize. The precipitate was filtered, washed and dried under vacuum. Crystalline form G of Cabazitaxel with all impurities below 0.10% (HPLC analysis) was obtained, yield 90%.

### EXAMPLE 3

Cabazitaxel anhydrous form B obtained as reported in WO 2009/115655 was dissolved in CH₃CN at temperature higher than 30°C. Subsequent to complete dissolution, the temperature was decreased to 20-25°C. The product crystallized and the slurry was filtered, washed with a mixture CH₃CN:H₂O 1:1 and dried under vacuum. Crystalline form G of Cabazitaxel with all impurities below 0.10% (HPLC analysis) was obtained, yield 84%.

### EXAMPLE 4

Crude Cabazitaxel was dissolved in CH₃CN at temperature higher than 30°C and, after complete dissolution, water (16 mL) was added dropwise keeping the temperature higher than 30°C. The product was left to crystallize at 20-25°C and the precipitate was filtered, washed with a mixture CH₃CN:H₂O 1:1 and dried under vacuum. Crystalline form G of Cabazitaxel with all impurities below 0.10% (HPLC analysis) was obtained, yield 90%.

### EXAMPLE 5

Cabazitaxel t-butylmethyl ether solvate (form II obtained as reported in WO 2012/142117, example 17) was dissolved in CH₃CN at temperature higher than 30°C. Some solvent was distilled under vacuum and after replacement of the distilled solvent with acetonitrile the temperature was decreased to 20-25°C. The product crystallized and the precipitate was filtered, washed with a mixture CH₃CN:H₂O 1:1 and dried under vacuum. Crystalline form G of Cabazitaxel with all impurities below 0.10% (HPLC A%) was obtained, yield 85%.

### EXAMPLE 6

The compound obtained according to Example 1, 2, 3, 4, 5 was characterised using the below described techniques.

### X-Ray Powder Diffraction

X-RPD patterns were collected on a Bruker D2-Phaser Diffractometer. The x-ray generator was operated at 30kV and 10 mA, using the CuKα line as the radiation source. The sample was packed on a suitable slit and the irradiated length was 10mm. Data were collected between 2 and 50 deg 2-theta with a step size of 0.02 deg 2-theta and a counting time per step of 3 sec.

The X-RPD pattern of form G shows a crystalline structure with distinctive reflections, expressed as 2-theta degrees values at approximately 7.6, 8.1, 8.8, 9.7, 10.1, 10.8, 11.6, 13.0, 13.5, 14.6, 15.1 and 16.0 ± 0.2.

Table shows the comparison between of X-RPD pattern of the crystalline form G of the invention and both the patterns of the anhydrous crystalline forms and hydrate forms described in W02009/115655.

**Table. XRPD comparison between anhydrous forms and hydrate forms reported in W02009/115655 and the crystalline form G of the invention**

| **Form G of the invention (crystallised from acetonitrile or acetonitrile/water)** | | **Application** W02009/115655 | | | | | | |
|---|---|---|---|---|---|---|---|---|
| | | **Hydrate forms** | | **Anhydrous forms** | | | | |
| | | **Mono-hydrate** | **di-hydrate** | **form B** | **form C** | **form D** | **form E** | **form F** |
| (°2 Theta) ±0.2° | Relative intensity | (°2 Theta) ±0.2° | (°2 Theta) ±0.2° | (°2Theta) ±0.2° | (°2Theta) ±0.2° | (°2Theta) ±0.2° | (°2 Theta) ±0.2° | (°2 Theta) ±0.2° |
| | | | | | | 3.9 | | |
| | | | 4.2 | | | | | |
| | | 4.3 | | | 4.3 | | | |
| | | | | | | | | 4.4 |
| | | 6.8 | | | 6.8 | | | |
| | | | 6.9 | | | | | |
| | | | | | | | 7.1 | |
| | | | | | | | | 7.2 |
| | | | | 7.3 | | | | 7.3 |
| | | 7.4 | | | 7.4 | | | |
| | | | 7.5 | | | | | |
| 7.6 | 37 | | | | | | | |
| | | | | | | 7.7 | | |
| | | | | | | 7.8 | | |
| | | | | | | 7.9 | | |
| 8.1 | 100.0 | | | 8.1 | | | 8.1 | 8.1 |
| | | | | | | | | 8.2 |
| | | | 8.4 | | | | | |
| | | 8.6 | | | | 8.6 | | |
| | | | | | 8.7 | | | |
| 8.8 | 6 | | | | | | | 8.8 |
| | | | | | | | 8.9 | |
| 9.7 | 2 | | | 9.8 | | 9.7 | | 9.6 |
| | | | 9.9 | | | | | |
| 10.1 | 18 | 10.1 | | | 10.1 | | 10.2 | 10.2 |
| | | | | 10.4 | | | | |
| | | | | | | 10.6 | | |
| 10.8 | 24 | | | | | 10.8 | 10.8 | |
| | | | 10.9 | | | | | 10.9 |
| | | 11.1 | | 11.1 | 11.1 | 11.1 | | 11.1 |
| 11.6 | 65 | | | | | | | |
| | | | | | | | | 11.2 |
| | | | 11.7 | | | | | |
| | | 11.9 | | | 11.9 | | | |
| | | 12.2 | | | | | | 12.1 |
| | | | 12.3 | | 12.3 | 12.3 | | 12.3 |
| | | | | | | | 12.5 | |
| | | 12.6 | 12.6 | | 12.6 | | | |
| | | | | 12.7 | | | 12.7 | 12.7 |
| 13.0 | 12 | | 13.2 | 13.1 | 13.1 | | 13.2 | 13.1 |
| 13.5 | 10 | | | | | | 13.4 | 13.1 |
| | | | | | | | 13.9 | |
| | | | | | | | | |
| | | | | 14.3 | | | | 14.3 |
| 14.6 | 9 | | | | | | | |
| 15.1 | 14 | | | | | | | |
| | | | | 15.4 | | | | 15.4 |
| 16.0 | 9 | | | 15.9 | | | | 15.9 |

### Dynamic Vapour Sorption (DVS)

The kinetic of moisture sorption at 25 °C is shown in Figure 5 (the corresponding isotherm plot is shown in Figure 6). The solid black line indicates the change in mass (referenced to the mass after drying) as a function of time; the dashed black line indicates the requested humidity in the sample chamber as a function of time.

In the initial drying step (360 min) there is the release of about 4.1 % of adsorbed moisture (Karl Fischer = 4.46%).

The equilibrium is reached in every single step and all the adsorbed moisture is completely released during the desorption stage, with the same kinetic of the sorption mechanism, in a completely reversible process.

In the second sorption/desorption cycle the sample shows the same behaviour.

There is no evidence of changes in the solid form G during sorption/desorption.

## Claims

1. A new crystalline form, referred to as form G, of Cabazitaxel of formula (I) 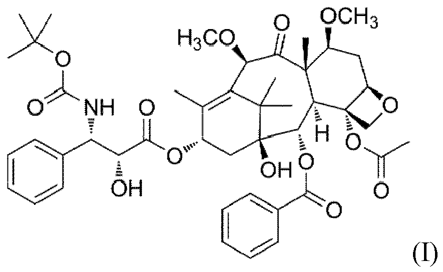 **characterised by** a x-ray powder diffraction pattern comprising distinctive reflections, expressed as 2-theta degrees [°] values, at approximately 7.6, 8.1, 8.8, 9.7, 10.1, 10.8, 11.6, 13.0, 13.5, 14.6, 15.1 and 16.0 ± 0.2.

2. The crystalline form according to claim 1, having a powder x-ray diffraction pattern substantially as depicted in Figure 1.

3. The crystalline form according to claims 1-2, for use as medicament.

4. The crystalline form according to claims 1-2, for use in the preparation of Cabazitaxel, Cabazitaxel salts and polymorphic forms thereof.

5. Pharmaceutical compositions containing the crystalline form G according to claims 1-2 in admixture with at least one excipient suitable for the pharmaceutical use.

6. The process for the preparation of the crystalline form G according to claims 1-2, comprising the recrystallization of Cabazitaxel from acetonitrile or from mixtures of acetonitrile and water.

7. The process for the preparation of the crystalline form G according to anyone of claims 1-2, comprising the following steps:
a) dissolution of Cabazitaxel in acetonitrile or acetonitrile/water at a temperature higher than 30°C;
b) decreasing the temperature to crystallize Cabazitaxel
c) filtration and drying under vacuum, affords the crystalline form G according to Claim 1.
